Europäisches Patentamt

(19) European Patent Office

Office européen des brevets

(11) Publication number: 0 249 502
A2

# (12) EUROPEAN PATENT APPLICATION

(21) Application number: 87305261.7

(22) Date of filing: 12.06.87

(51) Int. Cl.⁴: **C 12 P 21/00**
C 07 K 15/06, A 61 K 37/02,
A 61 K 49/00, C 12 N 5/00,
A 61 K 35/28
//(C12P21/00,C12R1:91)

(30) Priority: 12.06.86 US 873583

(43) Date of publication of application:
16.12.87 Bulletin 87/51

(84) Designated Contracting States:
AT BE CH DE ES FR GB GR IT LI LU NL SE

(71) Applicant: THE BOARD OF TRUSTEES OF THE LELAND
STANFORD JUNIOR UNIVERSITY
Stanford, CA 94305 (US)

(72) Inventor: Strober, Samuel
435 Golden Oak Drive
Portola Valley California 94025 (US)

Hertel-Wulff, Birgit
435 Golden Oak Drive
Portola Valley California 94025 (US)

(74) Representative: Goldin, Douglas Michael et al
J.A. KEMP & CO. 14, South Square Gray's Inn
London WC1R 5EU (GB)

(54) Soluble immune suppressor factor and suppressor cells.

(57) Natural suppressor (NS) cells secrete a soluble protein
(NS factor) which suppresses the mixed lymphocyte response.
Both NS and NS factor are useful in vivo to confer immunotoler-
ance with respect to allogeneic transplants, and to effect
immunosuppression.

**Description**

SOLUBLE IMMUNE SUPPRESSOR FACTOR AND SUPPRESSOR CELLS

Technical Field

The invention relates to suppressing immune response. More specifically, immortalized natural suppressor cells which themselves are capable of, and which secrete a soluble immunosuppressive factor capable of, eliciting immunotolerance in a host to coadministered foreign tissue and of donor immunocompetent cells to the tissues of the host are disclosed.

Background Art

"Immunotolerance" is a general term to describe the compatibility of materials which would be normally expected to result in an immune response. When tissues or cells are transplanted into an allogeneic host, absent an immunotolerant condition, the host immune system will mount an immune response to the foreign antigens (host v graft disease), and, more seriously, the immunocompetent cells in the transplant may respond to the antigens contained in the host (graft v host disease).

It has been known for over thirty years that neonatal mammals are capable of acquiring persistent immunotolerance with respect to allogeneic immunocompetent cells or to other antigenic substances administered within a few days after birth. For example, mice injected with allogeneic substances bearing the histocompatibility antigens are able later to accept skin grafts from donors of the previously injected genotype. More recently, it has been shown that this immunosuppressive property of neonates is simulated by adults subjected to total lymphoid irradiation (TLI), i.e., high dosages of radiation sustainable when nonlymphoid tissues are protected. Such radiation has been used in the treatment, for example, of Hodgkin's disease. Adult subjects who have been subjected to TLI are also capable of acquiring persistent immunotolerance to antigens administered within a few days of the completion of TLI.

The detailed mechanism by which neonates and TLI-treated subjects are capable of acquiring an immunotolerance with respect to antigens and cells administered within these windows is not understood. Both clonal deletion and active supression paradigms have been proposed. The picture is further complicated by the presence of at least two types of suppressor cells in neonatal spleens. One type is represented by macrophage precursors, which suppress in vitro the antibody response to sheep red-blood cells. This activity is inhibited by indomethacin, and the cells are thus presumed to be prostaglandin dependent. The other type is represented by null lymphocytes which inhibit the mixed leukocyte response (MLR). These cells are apparently prostaglandin independent. They lack the surface markers characteristic of T cells, B cells, or macrophages, and have morphologies similar to natural killer cells in that they are large granular lymphocytes which lack antigen specificity and which carry out their effective function without antigenic stimulation. This population of null suppressor cells has been designated, for purposes of symmetry with natural killer (NK) cells, natural suppressor (NS) cells (Oseroff, A., et al., J Immunol (1984) 132:101).

The NS cells found in neonatal mice spleens are also recoverable from the spleens of adult mice subjected to TLI and can be propagated in vitro under proper conditions indefinitely (Hertel-Wulff, B., et al, J Immunol (1984) 133:2791-2796, incorporated herein by reference). It has now been found that these cells are capable, when administered in vivo, of suppressing graft vs host disease initiated by simultaneously administered immunocompetent cells. In addition, the propagated cells secrete into supernatant media a soluble factor which is capable of suppressing the immune response as shown by the ability to suppress alloreactivity in the commonly used in vitro test (the mixed leukocyte reaction (MLR) mentioned above) and in vivo in suppressing the acute immune response referred to as graft vs. host disease.

The ability to suppress the capacity of immunocompetent donor cells to effect graft vs. host disease is of particular significance in view of the growing technology permitting successful allografts and organ transplants. Transfers of healthy tissues into recipients in need of them seems at present limited in the main by lack of immunotolerance with respect to the recipient. Thus, if the problem of graft-versus-host disease could be solved, the dangers associated with bone marrow transplants would be considerably reduced. In the case of whole organ transplants, rejection of the organ would be reduced.

It is well known that administration of allogeneic tissue in the form of bone marrow to either neonates or adults subjected to TLI during the "window" period will convert the recipient to a chimera, which will recognize as "self" both its own antigens and those of the alloantigen administered at this time. The chimeric character of the host is also such that subsequently introduced immunocompetent cells will not attack host tissue. These chimeras are not only specifically receptive to the simultaneously administered donor bone marrow, but also to other donor tissue. The chimeric recipient, therefore, will in the future be able to tolerate transplanted tissue from the original donor. The recipient normal adult host must, of course, be prevented from succumbing to an acute immune response effected by the administered bone marrow cells. The NS cells and the supernatant factors they secrete are capable of muting the immediate immune response sufficiently to permit the generation of characteristics of the chimera.

The cultured NS cells and the factors secreted by them are therefore useful in providing immediate immunotolerance in mammalian hosts by coadministration of the cells or factors along with foreign substances or tissues to which such immediate tolerance is desired. In addition, and in particular, these cells and/or factors permit the hosts to become tolerant of future grafts where the donor tissue is derived from the same donor as tissue coadministered with the cells or factor.

## Disclosure of the Invention

The invention provides immortalized cell lines capable of secreting factors useful in suppressing the immune response to any desired antigen. The immune response may be that of the host against allogeneic tissue (host versus graft) or that of donor immunocompetent cells to the tissues of the host (graft versus host). By using these cultured cells as direct means of production for the soluble factors, or as fusion partners in obtaining hybridoma lines, it is possible to obtain large quantities of natural suppressor factor protein (NS factor) capable of producing specific immunotolerance in the subject with respect to donor tissue from the same donor as that of coadministered allogeneic cells. This factor is useful in tissue transplant subjects so as to prevent immediate and future host-versus-graft and graft-versus-host disease. The NS cells can also be used directly in this way. In addition, it is possible to suppress autoimmunity by administration of NS cells or suppressor factor protein to the subject.

In one aspect, the invention relates to supernatants from immortalized cultures of natural suppressor (NS) spleen-derived cells, and to the natural suppressor factor protein they contain. While a murine-derived culture is described in detail, any mammalian spleen cells may be used. In other aspects, the invention relates to methods of inducing immediate immunosuppression and specific immunotolerance in mammalian transplant hosts, of inducing allogeneic immunotolerance in a recipient with respect to a future allograft, and to treating autoimmune diseases by use of the cells per se or of the natural suppressor protein secreted.

## Brief Description of the Drawings

Figure 1 shows the suppression of MLR by the NS cell supernatants.

Figure 2 shows the dose response curve for spleen enlargement as a manifestation of graft vs. host response in neonates.

Figure 3 shows survival rates of mice injected with allogeneic spleen cells with and without NS cells.

## Modes of Carrying Out the Invention

### A. Definitions

"Immunosuppression" is defined herein as that term is used generally to mean the general lowering of an immune response to challenge. While suppression of the response to a particular antigen may be desired, the suppression is not so limited.

"Specific immunotolerance" refers to a characteristic of a host described as the failure of an immune resonse to occur when a particular target antigen is encountered by either the host or donor material immunocompetent cells. The target antigen may be for example a simple soluble protein or carbohydrate, an infectious cell, or a cell surface. The donor tissue may be an allogeneic transplant tissue which contains immunocompetent cells, in this case the hosts' own tissue are target antigens.

"Donor material" refers to the material to which immunotolerance is desired. The nature of the donor material's immunoreactivity on a molecular level may or may not be known; for example, it may be desirable simply to tolerize a host against allogeneic transplants. In this case, the donor material consists of whatever immunogenic and immunocompetent substances are present in the transplanted tissue.

It is desirable to prevent the response of the host cells to donor tissue (host-versus-graft disease) and of graft-versus-host disease. The latter is accomplished by preventing an immune response on the part of a transplant tissue to the tissues of the host. Under such circumstances, the "donor material" comprises immunocompetent cells which respond to whatever antigenic substances are present in the transplant recipient which trigger an immune response by this material.

"Null surface phenotype" refers to the property of the cells of the invention wherein they lack the surface markers characteristic of T cells, B cells, or macrophages, and have morphologies similar to natural killer cells. Thus, they are large granular lymphocytes which lack antigen specificity and carry out their functions without antigenic stimulation. With regard to surface markers, the appropriate surface markers in the murine system include Lyt-1, Lyt-2, and surface immunoglobulins such as murine Ig and Ia. Each mammalian system has its typical corresponding markers, which in the human system includes markers recognized by various monoclonal antibodies known in the art. The corresponding human cells would be expected to be OKT4+, OKT8±, LEU3−, LEU11+, and LEU7±.

"Corresponding" target cells refers to standard assay cells which cytotoxic lymphocytes would be expected to lyse. In the murine system, these are YAC-1. In the human system they are K562 cells.

"Cell line" or similar words such as "cells", "cell cultures", and the like refer to the specific genotype described, to its progeny, and to mutants and derivatives thereof which retain the essential characteristics of the original cell. It is well understood that naturally occurring or deliberately induced mutations are common in propagation of particular cell lines, many of which are irrelevant to the desired properties. For example, in the cells of the invention, the ability to suppress the mixed lymphocyte reaction must be retained, as well as the characteristic of expressing the unique surface receptors characteristic of neither T nor B cells. Other metabolic properties, such as nutritional requirements, antibiotic resistance, and the like are irrelevant to the functionality of the cells, and the definition, as it relates to a particular "cell line", includes mutants and derivatives which contain such alterations. In addition, it is understood to be possible to hybridize two cell lines to retain desired characteristics from both partners, eliminating others. Such hybridomas are considered to be derivatives of the original cell line when the required properties are retained.

"Suppresses the mixed lymphocyte reaction (MLR)" refers to the ability of the subject material (soluble factors or cell line) to prevent the uptake of a marker substance such as thymidine by maturing T cells in the response which occurs when lymphocytes from allogeneic sources are mixed. Specifically, this property may be tested by the procedure specified according to Hertel-Wulff et al (supra), incorporated herein by reference (see below).

"Simultaneous" administration is meant to be approximately contemporaneous, i.e., within several hours.

B. Assays

The NS cells and NS factor protein of the invention are characterized by, and are useful by virtue of, their in vitro or in vivo activities. Their in vitro activity is demonstrated using the mixed leukocyte reaction (MLR), a commonly used protocol to detect alloreactivity. In this protocol, lymphocytes from two genetically different individuals are mixed in a culture medium. As the cells of each individual bear different major histocompatibility antigens (MHC) on their surfaces, the T cells of each respond to these differences. The measurement of the response can be simplified by irradiating the cells of one species to permit them to behave only as stimulators of an immune response by the unirradiated (responder) cell population. The immune response is presumed to involve initial secretion of lymphokines by amplifying T cells among the responders, which lymphokines mediate the maturation of cytotoxic T responder cells. This proliferation is monitored by uptake of labeled thymidine ($[^3H]$ TdR) and suppresion of the response is indicated by inhibition of labeled thymidine uptake.

In more detail, as the assay was conducted herein, $5 \times 10^5$ normal BALB/c spleen responder cells were mixed with $7.5 \times 10^5$ normal C57BL/Ka stimulator cells which had been irradiated in vitro. After 4 or 5 days, the mixed culture was pulsed with $[^3H]$ thymidine and thymidine uptake measured after 18 hr. Control cultures typically gave $110,000 \pm 7,000$ cpm, and this measure was compared with cpm obtained from cultures to which NS cells or supernatant-containing NS factor had been added, as described below. Both the immortalized NS cells and the supernatant media from cultures of these NS cells are capable of inhibiting thymidine uptake in the MLR.

The ability to inhibit this uptake is independent of the antigenic makeup of the stimulator cells and does not require that the haplotypes of the suppressor and responder cells be matched. The mechanism of the suppression is not known, but it has been established that neither IL-2 induced proliferation of HT-2 cells nor IL-1 secretion by macrophages is inhibited.

To assess the in vivo activity for suppression, mice which have been irradiated with sublethal dosages of radiation are injected with suspensions of the NS cells or with their supernatants, each coadministered with spleen cells or bone marow derived from an allogeneic species. Subsequent prevention of lethal graft vs. host disease confirms the immunosuppressive action with respect to the donor cell immune attack on the host. Both the NS cell suspensions and the NS factor proteins are able to suppess the immediate immune response of the donor cells. For those recipients receiving bone marrow rather than spleen cells, immunotolerance to later skin allografts where the donor was of the strain from which the bone marrow was derived was achieved in the recipients. These direct experimental results demonstrate the ability of the NS cells and the NS factor to permit immunotolerance. While these data show the prevention of graft-versus-host disease--i.e., immunotolerance of donor immunocompetent cells with respect to host tissue, it thereby follows that the host's own immunocompetent cells are, in a complementary manner, tolerized to allogeneic or other foreign tissue. In addition, these, results demonstrate the ability of the cells and factor to generate immediate immunosuppression.


C. Propagation of the NS Cells

Propagation of spleen cells derived from TLI or neonatal mice was conducted as described by Hertel-Wulff, et al (supra). Mice were used as convenient subjects, but, of course, other vertebrates could also be used.

Human cells are also available as subject cultures using spleens removed for medical indications from neonates or removed from previously ascertained donors who, for other medical indications, had been subject to total lymphoid irradiation, and are recently deceased. In the alternative, human bone marrow which carries null phenotypes can be used. If bone marrow is used, the null lymphocyte population is isolated using standard Ficoll-Hypaque gradients and cultured in standard tissue culture media with the addition of an appropriate lymphokine such as IL-2 or the supernatant from human peripheral blood lymphocytes stimulated with PHA. The null phenotypes are propagated as a polyclonal population, and then colonies are analyzed for suppressive activity using a human mixed lymphocyte reaction (MLR) similar to that described for the murine cells herein. The isolated phenotype resembles that described herein for the murine system. In addition to suppressing the appropriate MLR, the cell has a null phenotype and no natural killer function--i.e., it is not able to .lyse human tumor K562 cells.

Briefly, for the murine system exemplified, spleens were removed aseptically either from newborn BALB/c mice (1-14 days old) or mice which had been subjected to TLI. In the irradiation procedure, 4-6 month old male BALB/c mice were anesthetized daily with pentobarbitol and were positioned in an apparatus designed to irradiate the major lymphoid organs (lymph nodes, spleen, and thymus) described by Slavin et al. J Exp Med

(1977) 146:34; skull, lungs, tail, and hind legs were shielded. The mice were given 200 rad/day 5 times a week for a total dose of 3,400 rad, using a dose rate of 92 rad/min with a 0.35 mm copper filter and a 52 cm source/axis distance. Tetracycline was added to the drinking water during TLI and for one week after completion of radiation. TLI-treated mice were sacrificed between 1-3 days after completion of TLI.

Single cell suspensions were prepared by incising the capsule and disrupting the splenic parenchyma. The suspended cells were cultured in RPMI 1640 containing 25 mM HEPES, 2 mM glutamine, and $5 \times 10^{-5}$ M 2-mercaptoethanol, with 10% fetal calf serum (FCS) and 10% supernatant from Conconavalin A stimulated rat spleen cells (CAS). CAS was prepared as described by Oseroff, A. et al, J Immunol (1984) 132:101-110. Aliquots of $1.5 \times 10^6$ cells per well were placed into 24-well plates, and aliquots of $5 \times 10^6$ cells were placed in small flasks. In some cultures $5 \times 10^5$ TLI-treated cells were incubated with $1 \times 10^6$ spleen cells irradiated with 1500 rads in vitro, as feeder cells, and fresh feeder cells were added every 10-14 days for the first 2-3 weeks. Cultures were fed with 10% CAS-containing medium every 2-3 days for the first 2 months, at which time the cultured cells grew slightly adherent to the plastic surface. Cultures were then fed every day and maintained in vitro for at least 48 months. After 8 months in cultures, the cells were cloned using limiting dilution by seeding using 1.5-5 cells per ml in complete medium supplemented with 10% CAS.

The cloned cells were moderately large with granular and vacuolated cytoplasm. Two cell lines, one obtained from neonates and designated 4BA4, and the other obtained from TLI mice and designated TLI-2.4C, were used for further study.

The cultured cells are induced to produce the soluble suppressor factor by addition of suitable inducing agents. One effective class of such agents includes those which activate the phosphatidyl inositol pathway, such as the phorbol esters. For example, secretion of the NS factor is stimulated by the addition of 5-20 ng/ml of PMA and 0.05-1.0 μg/ml of a calcium ionophore to the medium.

Similar conditions are used to stimulate factor production from other mammalian, including human, cell cultures.

## D. Characterization of Cultured NS Cell Lines and Induction of Factor

NS cells are unusual with respect to surface markers. They have been shown to express the Thy-1 antigen but not Lyt-1 and Lyt-2 antigens nor surface immunoglobulins found on T and B cells respectively, and do not carry the macrophage markers identified by the monoclonal antibodies anti-MAC-1 and F4/80. They do not stain for nonspecific esterase, and do not adhere to plastic or glass. Human-derived cells have similar properties with respect to the appropriate markers.

Though similar in surface phenotype to NK cells, NS cells are not able to lyse YAC-1 tumor cells. (The appropiate line for human cells is K562.) However, they suppress [$^3$H]TdR uptake by a myoglobin responsive T-cell line in the presence of myoglobin.

The cell lines of the invention can generally be derived and cultured as described above. They can be further immortalized by fusion to immortalizing cell lines, infection with virus, or other means known in the art. The soluble factor is produced by suitable inducing conditions, which include those particular conditions specified above, as well as addition of other materials which stimulate the phosphatidyl inositol pathway to the medium. The supernatants containing NS factor may be used per se, or may be subjected to standard purification techniques to isolate the factor with the suppressor activity by tracking active fractions as measured by suppression of the MLR.

## Characterization of the Suppressor Factor

The suppressor factor from the murine cells exemplified has been partially characterized. The analogous factor from cells of other mammalian species, including humans, has similar properties. First, it has been verified that the supernatants of the isolated cell lines are free of activity exhibited by IL-1, IL-2, IL-4, tumor necrosis factor (TNF), and TGF-β. The supernatants do contain IL-3 and γ-interferon activity. However, the suppressor factor has been shown not to be identical to these. With respect to IL-3, recombinant IL-3 does not show suppressive activity in the standard MLR. Recombinant γ-interferon does show such activity; however, after removal of all γ-interferon from the supernatant by immunoaffinity chromatography, the suppressive activity of the supernatants is retained.

Chromatographic separation on Sephadex G-150 of the proteins from the media in which the cells were cultured showed 90% of the suppressor activity in the two peaks of 135 kd and 240 kd. These peaks are removed from the peaks associated with elution of the major proteins in the media. In an examplary procedure, 650 μl of the supernatant is loaded onto a Sephadex G-150 column and eluted in pyridine:acetic acid buffer or PBS buffer at pH 7.2. The eluate is collected in 500 μl aliquots using size markers.

The specific activity of the pure suppressor factor is clearly quite high, as the protein profiles on the Sephadex column appear identical for induced and noninduced cells; however, only the 135 kd and 240 kd peaks from the induced cultures show suppressive activity.

## E. Suppressor Activity

## E.1 Suppression of MLR

Both the NS cells and their induced supernatants were capable of suppressing the MLR conducted as follows: Responder cells ($5 \times 10^5$) and stimulator cells ($7.5 \times 10^5$) were incubated with graded numbers of the NS cells in 0.3 ml/wells in 96-well microculture plates. The culture medium was supplemented with 2 mM glutamine, $5 \times 10^{-5}$ M 2-mercaptoethanol, 100 units/ml penicillin, 100 µg/ml streptomycin, and 10% pooled human serum (VSP, Biocell Laboratories, Carson, California). The NS cells and stimulator cells were given 3300 rad before incubation. Cultures were maintained at 37°C in 5% $CO_2$ for 5-6 days. Eighteen hr before termination, 1/µCi of [³H]TdR (6.7 Ci/mM) was added to each culture. Cells were harvested with a semiautomatic cell harvester and counted in a Beckmann liquid scintillation counter.

Three illustrative cell lines cultured from TLI mice, designated TLI-2.B7, TLI-2.H5, and TLI-2.4C, suppressed the MLR significantly at co-cultured cell counts of $2 \times 10^4$ per well. Suppression in the range of 90% was obtained at this concentration when C57BL/Ka stimulator and BALB/c responder cells were used. Similar results were obtained with A/J responder and C57BL/Ka stimulator cell lines.

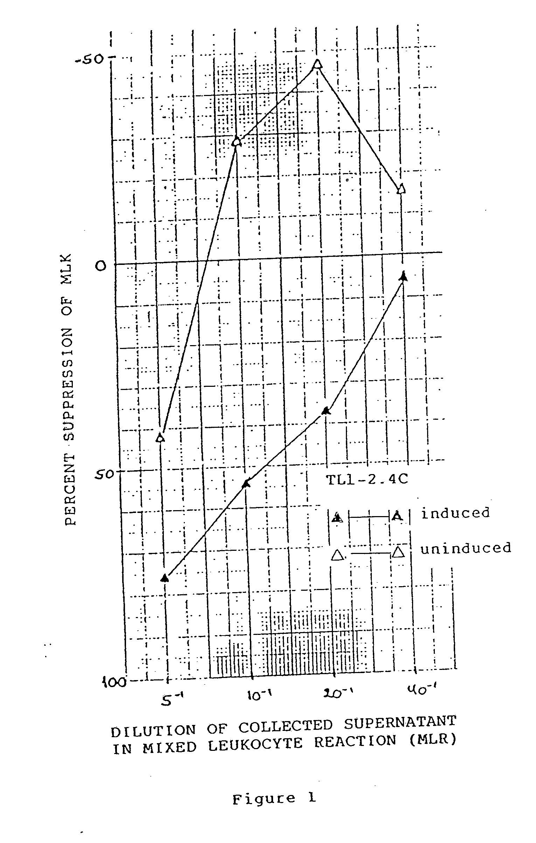

Suppression of the mixed lymphocyte reaction was also obtained when supernatant from TLI-2.4C cells was added to the cultures (controls give 110,000 ± 7,000 cpm). Figure 1 shows the data obtained in the MLR comparing the result for added supernatant with and without induction.

Supernatants were obtained from cloned TLI-2.4C cells 24 hr after they had been induced with 10 ng/ml PMA (4-phorbol-12-myristate-13-acetate) and 0.26 µg/ml A23187 calcium ionophore for 4 hr at 37°C/5% $CO_2$. When dilutions of this induced supernatant were added to the MLR, a 75% suppression of the MLR was obtained using a 1:5 dilution of the supernatant. Uninduced supernatant gave only a 40% suppression at this dilution. The results are more clearly seen at a 1:10 dilution, where the induced supernatant gives a suppression of 55% while uninduced supernatant shows a 30% stimulation of the reaction.

The activity of the induced supernatant is destroyed by pronase treatment and is associated with a dialysate of >20 kd.

### E.2 In Vivo Assays

#### Suppression of Spleen Enlargement

When foreign immunocompetent cells are supplied in sufficiently small amounts relative to the state of immunosuppression of the host, the foreign (donor) cells are generally not lethal to the host, but the host displays a measurable response in the form of spleen enlargement. An assay for graft-versus-host disease based on this observation was disclosed by Simonsen, M., Prog Allergy (1962) 6:349-467. This assay for sublethal graft-versus-host disease was used to determine the effect of TLI-2.4C and 4BA4 cell lines on this response.

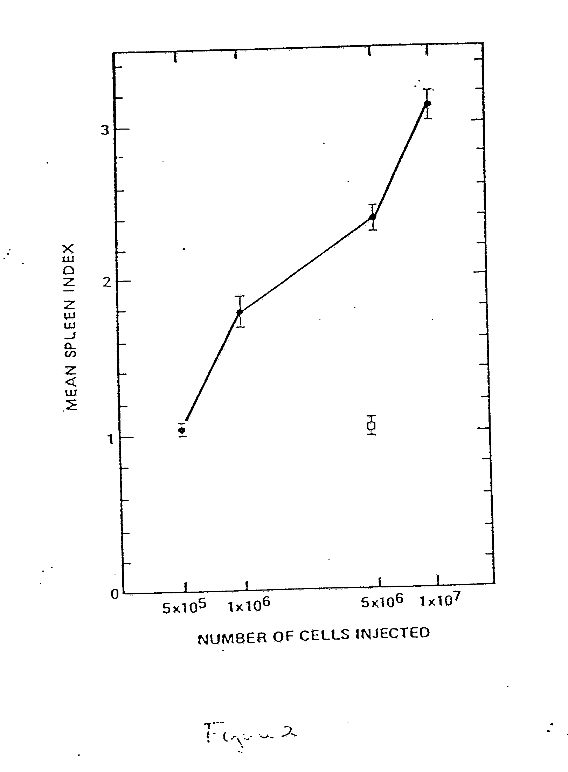

To determine the proper dosage level for the donor cells, 0.5, 1, 5, or $10 \times 10^6$ adult C57BL/Ka spleen cells were injected into the FI cross BALB/c x C57BL/Ka neonatal hosts. The adult (8-12 weeks old) dissociated spleen cells were prepared in tissue culture medium RPM1-1640 (GIBCO, Grand Island, NY) and injected intraperitoneally in 0.1 ml into the FI cross on day 4-7 after birth. Eight days later recipient spleens were removed and assayed. Injection of $5 \times 10^6$ cells was shown to give an easily measurable response of the order of a 2.4-fold (average) increase in spleen size, and was on the dose responsive portion of the curve obtained, shown in Figure 2.

Spleen indices were calculated and represent the ratio of the weight of the spleens of injected mice to the weights of uninjected litter mate controls. Indices greater than 1.0 are indication of graft vs. host disease.

Table 1 shows the results obtained when the subject neonates were injected intraperitoneally with $5 \times 10^6$ C57BL/Ka spleen cells 4-7 days after birth with and without NS cells or control HT-2 cells and the spleen indices measured 8 days later. As expected, controls injected with $5 \times 10^6$ FI hybrid spleen cells showed no spleen enlargement.

## Table 1

### Spleen Index

(Mean)

| | |
|---|---|
| No addition | 2.6 |
| +15x10$^6$ TLI-2.4C | 1.2 |
| No addition | 2.7 |
| +15x10$^6$ 4BA4 | 1.8 |
| No addition | 2.5 |
| +15x10$^6$ HT-2 | 2.3 |
| No addition | 2.6 |
| +5x10$^6$ TLI-2.4C | 1.6 |
| No addition | 2.5 |
| +5x10$^6$ HT-2 | 2.3 |

The results are clear that co-injection of either TLI-2.4C or 4BA4 cells at 15 x 10$^6$ cells is effective in suppressing graft vs. host disease as measured by the spleen enlargement caused by the foreign cells. Reducing the NS cells injected to 5 x 10$^6$ reduced the amount of suppression but did not destroy it. A control T-cell line (HT-2) does not suppress.

Suppression of Lethal Graft-Versus-Host Disease

When similar administrations of foreign tissues are made to irradiated weanling hosts, the response is not limited to spleen enlargement, and the injections are generally fatal. Previous studies have shown that sublethally irradiated adult BALB/c mice injected IV with C57BL/Ka spleen cells are killed within two weeks. This is in part due to the high concentration of T cells in the spleen.

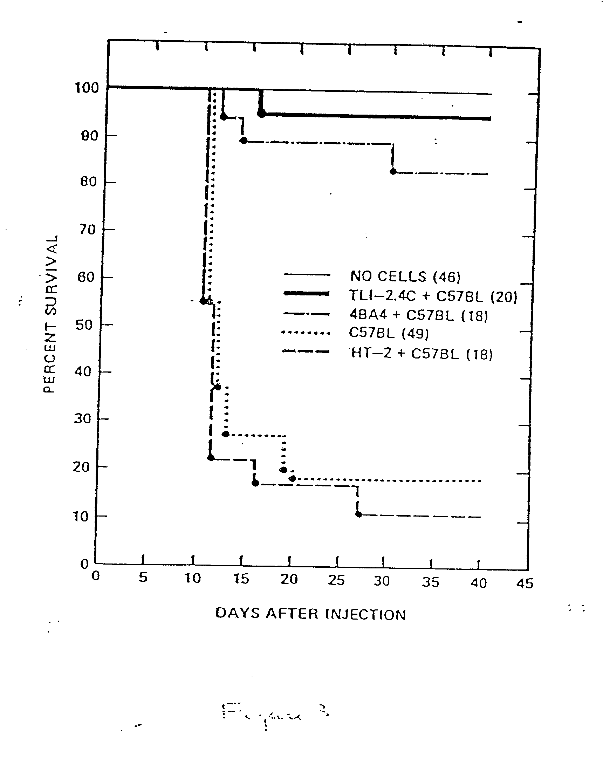

BALB/c weanlings were given 400 rad whole body irradiation 6-12 hours before administration of 5 x 10$^6$ C57BL/Ka spleen cells in 0.5 ml RPMI1640, either alone or in combination with the NS cells. Under these circumstances, BALB/c 21 day old weanlings injected intraperitoneally with C57BL/Ka spleen cells were killed (85% die by 30 days). As would be expected, C57BL/Ka weanlings similarly treated survive. However, for BALB/c mice receiving 15 x 10$^6$ cloned NS cells (TLI-2.4C) co-injected with the C57BL/Ka spleen cells, only 5% of the hosts died after 30 days. Co-injections of 15 x 10$^6$ 4BA4 cells are only slightly less effective. Co-injection with HT-2 cells gave results similar to those of the controls. These results are summarized in Figure 3, where the numbers in parentheses show the number of mice in each group. The survival rates at 100 days were the same as those shown for 40 days in the Figure. Those few mice receiving co-injected HT-2 cells which did survive were runted.

It is clear from the foregoing results that the cloned NS cells are capable of suppressing the acute graft-versus-host disease mounted against immunocompromised hosts. (It should be noted that intraperitoneal injection of the spleen cells and of the NS cells was essential; similar experiments conducted intravenously were not successful, possibly due to failure of NS cells to migrate to the host spleen and interact with the donor cells.)

The conditions of the in vivo treatment performed above, however, did not result in the NS-cells conferring chimeric characteristics on the host. To demonstrate this, peripheral blood mononuclear (PMN) cells were assessed for the presence of C57BL/Ka donor cells. PMN were isolated from surviving hosts after 30 days of test period, and incubated with anti-C57BL/Ka antiserum with complement in a microcytotoxicity test performed as described by Slavin, S., et al, J Exp Med (1977) 146:34-48. Where C57BL/Ka characteristics present in any PMN cells, cell death would have resulted; however, none was observed. Also, these hosts were not capable of accepting C57BL/Ka skin grafts within 2 wk after the initial injection.

E.3 NS Cells Are Permissive to Establishment of Chimeric Nature and Immunotolerance

While the NS cells were shown to protect subjects against the acute response produced against injection of allogeneic spleen cells, these spleen injections failed to confer the desired immunotolerance and chimerism on the host. This failure may be due to the nature of the injected tissue and thus its failure to repopulate the host bone marrow and spleen with C57BL/Ka hematopoietic stem cells. Spleen cells contain a high population of T-lymphocytes, thought to be responsible for the acute graft-versus-host disease, but a relatively low population of stem cells, which are relatively undifferentiated and are thought to be responsible for, or at 1east essential to, production of immunotolerance.

Accordingly, procedures similar to those above were conducted using bone marrow rather than spleen cells as the source of foreign tissue. Adult BALB/c mice were given lethal whole body irradiation (700 rad) one day before intraperitoneal injection of 50 x $10^6$ C57BL/Ka bone marrow cells; with or without 15 x $10^6$ TLI-2.4C cells. 12 of 14 mice survived 30 days later without evidence of graft-versus-host disease when the NS cells were co-injected. Among controls given no NS cells, 8 of 10 survived.

PMN from all surviving animals were tested by the microcytotoxicity assay as above and found to be chimeras as shown by 95% killing of PMN cells. Further, they were able to accept C57BL/Ka, but not C3H, skin grafts after 40 days.

The spleen cells of the chimeras were further tested for their ability to induce graft-versus-host disease in fresh BALB/c mice recipients. Normal C57BL/Ka donor cells, when injected at 10 x $10^6$ spleen cells intraperitoneally in 0.5 ml RPMI 1640 medium into either BALB/c or C3H recipients result in the death of all mice injected within 14 days. As would be expected, control mice having no cells injected survived. Spleen cells of the chimeras, injected similarly into BALB/c or C3H mice, induced graft versus host disease only in the C3H recipients; the BALB/c recipients survived injection of the chimeric spleens whether derived from chimeric donors given a C57BL/Ka bone marrow injection alone or in combination with NS cells. The immunotolerance conferred on the chimeric donors was specific to BALB/c recipients.

## F. Use of NS Cells or NS factor to Confer Immunotolerance

The NS cells and induced supernatants as well as the NS factor protein per se of the invention will be useful in conferring immunosuppression and immunotolerance on a host subject. Subjects susceptible to this treatment include any vertebrate species, including human, but particularly the NS wells and NS factor compositions are adaptable to use in mammals. The conferred immunosuppression or immunotolerance is especially useful in permitting the host to accept simultaneous or future transplants of tissues from an allogeneic donor. The dosage levels required are highly dependent on the nature of the host and on the nature of the immunological challenge. However, as an overall estimate, in the method of the invention, the NS cells are administered to the host in the amount of approximately $10^8$-$10^{10}$ cells/kg of host weight, along with a comparable number of cells to be used in the allogeneic transplant. The amount of supernatant NS factor administered is comparable, i.e., the amount produced in the supernatant from about this same number of induced cells. Coadministration with similar numbers of foreign immunocompetent cells permits contemporaneous acceptance of these transplants. In addition, the foregoing treatment may be used in conjunction with subsequent implantation or injection of tissue from the same donor which has been co-injected with the NS or NS factor, as above. The initial simultaneous administration may use immunocompetent donor tissue, such as bone marrow or spleen cells, preferably bone marrow.

Administration is typically by injection, either intravenous (especially for the soluble factor) or intraperitoneal (which is preferred for the NS cells). However, other modes of administration, such as oral, transmucosal, or using other formulations as is understood by those in the art may also be used.

## Claims

1. Natural suppressor factor protein.

2. Natural suppressor factor protein for use in a method of treatment of the human or animal body by surgery or therapy or in a method of diagnosis practised on the human or animal body.

3. A process for producing natural suppressor factor protein comprising culturing cells of null surface phenotype which display no natural killer activity against corresponding target cells and recovering natural suppressor factor protein from culture medium.

4. A process according to claim 3 wherein the cells are spleen cells or bone marrow cells of a mammalian neonate or irradiated mammal.

5. A process according to claim 3 wherein the cells are from an immortalised cell line derived from spleen cells or bone marrow cells of a mammalian neonate or irradiated mammal.

6. A process according to any one of claims 3 to 5 comprising recovering protein fractions having molecular weight of about 135kd or about 240kd or both.

7. A process according to any one of claims 3 to 6 comprising production of natural suppressor factor protein by activating the phosphatidyl inositol pathway of the cells.

8. Cells of null surface phenotype which display no natural killer activity against corresponding target cells for use in a method of treatment of the human or animal body by surgery or therapy or in a method of diagnosis practised on the human or animal body.

9. Immortal cells capable of secreting natural suppressor factor protein derived from spleen cells or bone marrow cells of a mammalian neonate or irradiated mammal.

10. Immortal cells according to claim 9 for use in a method of treatment of the human or animal body by surgery or therapy or in a method of diagnosis practised on the human or animal body.

11. A composition comprising natural suppressor factor protein according to claim 1 or claim 2 or cells according to any one of claims 8 to 10.

12. A pharmaceutical composition comprising natural suppressor factor protein according to claim 1 or claim 2 or cells according to any one of claims 8 to 10 and a pharmaceutically acceptable diluent or carrier therefor.

13. A composition according to claim 11 or claim 12 which is, or is derived from culture medium in which cells of null surface phenotype which display no natural killer activity against corresponding target cells have been cultured.

14. A composition according to any one of claims 11 to 13 for use in a method of treatment of the human or animal body by surgery or therapy or in a method of diagnosis practised on the human or animal body.

15. Use, in the production of a medicament for use in a method of treatment of the human or animal body by surgery or therapy or in a method of diagnosis practised on the human or animal body of natural suppressor factor protein according to claim 1 or claim 2 or of cells according to any one of claims 8 to 10 or of a composition according to any one of claims 11 to 14.

Figure 1

Figure 2

Figure 3